# EUROPEAN PATENT APPLICATION

(11) **EP 1 586 305 A1**
(43) Date of publication of application: **19.10.2005**
(21) Application number: 05075777.2
(22) Date of filing: 05.04.2005
(51) Int. Cl.: A61K 7/42, A61K 7/48, A61K 7/06, A61K 7/02

(54) **Oily cosmetic composition having a suspensory capacity**

(30) Priority: 07.04.2004 BR 0400438
(71) Applicant: Botica Comercial Farmacêutica Ltda., Sâo José dos Pinhais PR 8306 5-620 (BR)
(72) Inventor: Mazer, Ronit, Merces Curitiba PR (BR); de Oliveira Praes, Carlos Eduardo, Uberaba, Curitiba, PR (BR); Oliveira Mendes, Tania Regina, Downtown, Sao Jose dos Pinhais, PR (BR); Patriarca, Paola Maria, Curitiba, PR (BR); Feferman, Israel Henrique S., Sao Jose dos Pinhais, PR (BR)
(74) Representative: Carvajal y Urquijo, Isabel

(57) **Abstract**

OILY COSMETIC COMPOSITION HAVING A SUSPENSORY CAPACITY, oily cosmetic composition acting as a lubricant, moisturizer and skin protector, mucosas and hair, being further in condition to act as a basis for decorative or fragrant cosmetics. This composition is exhibited in the form of oil-gel, which may be transparent, translucent or opaque, and exhibits as a different aspect an excellent suspensory capacity, combined with an easy application, good sliding on the skin and a pleasant and silky sensorial after the application. This set of features is obtained through the association of a block copolymer, composed of styrene-ethylene/propylene with silica, on an oily basis. The composition may contain particles in suspension, with decorative and/or functional properties.

## Description

The present invention patent relates to oily cosmetic preparations for the treatment, protection and/or decoration of the skin, mucosas or hair. This composition is exhibited in the form of oil, oil-gel, paste or pomade and may be transparent, translucent or opaque. Its distinctive properties concern an excellent suspensory capacity of particles in addition to its sensory properties, such as easy sliding, emollient and silky touch on the skin, mucosa and hair. The perception of the product is pleasant and distinct from the traditional thickened oily cosmetic compositions, containing particles in suspension, through its features, such as high dispersibility, emollient properties and low stickiness.

The oily cosmetic preparations constitute one class of cosmetic products for affording hydration, protection, treatment, decoration and for perfuming the skin, mucosas and hair. The oils are rather used for corporation applications and solar protectors, as they exhibit good sliding, dispersibility and emollient properties, acting as moisturizers, its use being quick and practical. However the thickened oily formulations, particularly, are useful nowadays mainly as a basis for the labial make-up products, such as the labial brightness. This is because these compositions, usually, exhibit a certain stickiness, owing to the presence of ingredients acting as suspension agents of pigments, and this feature is not acceptable for the products intended to be applied on the skin or hair. The applications of thickened oily formulations to the skin and hair are limited, in view of the difficulties to formulate products having an adequate sliding and low stickiness. Differently from the cosmetic fluids, a thickened oily formulation having a suspensory capacity allow the incorporation of particles and the consequent incorporation of protective agents or the decoration of the skin, mucosas or hair.

The thickened cosmetic oily compositions for application to labial make-up (labial brightness) are often composed of silica and high viscosity oils. These ingredients enable the maintenance of the pigments in suspension, however they provide sticky sensorial and of a low dispersibility. Silica is often used for thickening oily compositions, however in higher concentrations, and, in order to obtain an oil-gel, it exhibits a not so elegant sensorial and it is difficult to be spread and leaves an extremely dry and disagreeable touch on the skin. Another form to obtain an oily gel is making use of copolymers of styrene-ethylene /propylene block, which, in certain determined concentrations, give rise to viscous and transparent gels, with good sliding features and low stickiness. However, under these concentrations, its suspensory capacity is insufficient. Its suspensory capacity may be improved by excessively increasing the concentration of this polymer, however it loses its agreeable sensorial, making the gel heavy, of a difficult dispersion and of a high stickiness.

In this area, there is the particular interest in the development of thickened oily formulations showing good suspensory capacity of particles and that, at the same time, exhibiting good features of dispersibility, sliding and emollient properties, however without stickiness, leaving a pleasant sensorial on the skin, the mucosas or hair. Moreover, the product is not to cause allergic reactions or irritation on the skin and mucosas, which would make unfeasible its use. To combine these features is the built-in art in the development of this type of product.

Document EP0497144b1 protects a composition containing a copolymer of styrene-ethylene-propylene block, combined with one or more emollient properties and with one or more dying agents or solar blockers. It shows, as greater objectives, the silky sensorial left on the skin, the easiness of sliding and the features of not forming flakes after the application on the skin. Differently from this invention, it makes no reference to the suspensory capacity of the block copolymer and its combined use is not extended to another auxiliary agents of rheology, such as silica.

Patent US6312672B1 protects the application of butadiene, isoprene and/or styrene copolymer in solar protector formulations, such as emulsions, with a view to improving the resistance to the water of the products. Differently from this invention, its object is neither the formulation of oily gels having a suspensory capacity, nor it mentions the combination of the copolymers with rheology auxiliaries.

Document US608083516 discloses a cosmetic composition having a fixation feature and dying durability on the skin and hair, by using a styrene-ethylene/propylene block copolymer, either combined or not to an alkyl cycloalkylacrylate. Differently from this invention, it does not mention the use of the copolymer for thickening and suspending particles in anhydrous oily formulations in combination with silica.

Patent US20030147931 protects the use of trimethylated silica for improving the durability and minimizing the transfer of cosmetic compositions. Differently from this invention, it does not provide for the association of the silica with other suspensory or thickening agents with a view to improving the sensorial associated with a good suspensory capacity.

The instant invention patent discloses an oil-gel composition, paste or pomade for application on the skin, mucosas or hair, which may be transparent, translucent or opaque, with a capacity of adequately keeping particles in suspension, at the same time exhibiting easy sliding and emollient properties, without any stickiness. The formulation of this invention contains a high concentration of conventionally oily components used for obtaining fluid corporal oils, such as, for instance, mineral oil and fractions thereof and derivatives, silicones, esters and cosmetic ethers, "Guerbert" alcohols, fatty acid triacylglycerals and vegetable oils. These exhibit a high capacity for retaining humidity on the skin and constitute the basis of the composition of this invention. Being combined on the compound basis by cosmetic oils, this composition exhibits two essential components for the result of obtaining a gel-oil having a capacity of maintaining particles in suspension and the good features of application and sensorial. The first one of these components is a hydrogenated block copolymer of styrene-ethylene/propylene, or, further, styrene/isoprene halogenated copolymer. This linear copolymer exhibits an elastic and flexible structure, being composed of styrene at a concentration between 30% and 40% in mass. It exhibits a type-S-EP structure, wherein "S" denotes a block comprising styrene monomers and "EP" denotes a block comprising ethylene and propylene monomers. The polymer, by way of example, has been tested as to its capacity of suspending teraphthalate particles of polyethylene, commonly used in cosmetic products within the following base formulation:

| Ingredients | Essay 1 | Essay 2 | Essay 3 | Essay 4 | Essay 5 |
|---|---|---|---|---|---|
| Styrene-ethylene/propylene copolymer | 6.0% | 7.0% | 8.0% | 9.0% | 10.0% |
| Mixture of Cosmetic Oils | Qsp. 100% | Qsp. 100% | Qsp. 100% | Qsp. 100% | Qsp. 100% |
| Polyethylene Terephthalate | 0.5% | 0.5% | 0.5% | 0.5% | 0.5% |
| Suspensory capacity (50° C) | Insufficient decantation over a time less than 24 hours | Insufficient decantation over a time less than 24 hours | Insufficient decantation over a time less than 07 days | Insufficient decantation over a time less than 7 days | Satisfactory No decantation over the time of 7 days. |
| Sensorial | Easy Dispersion Sensorial Emollient with low stickiness. | Easy Dispersion Sensorial Emollient with average stickiness. | Average Dispersion Sensorial Emollient with average stickiness. | Difficult Dispersion Sensorial Emollient with high stickiness. | Difficult Dispersion Sensorial Emollient with high stickiness. |

After these preliminary essays, one could see that, by combining the sensorial features, such as easy dispersion, emollient sensorial and low stickiness with a good suspensory capacity, that would not be possible by making use of only the styrene-ethylene/propylene copolymer. With the minimal amount for obtaining a good suspensory capacity (10,0%), taking into consideration the term of 7 days at the temperature of 50°C, the product sensorial was highly affected.
The second of these components is silica. The term silica relates to the silicon bioxide compounds, SiO₂, in the various forms, including crystalline vitreous and amorphous silicas. The amorphous silica is also known as pyrogenic silica, or colloidal silica and may be obtained by hydrolysis of chlorinesilanes at a high temperature reaction. It concerns a fine and amorphous white powder exhibiting a high affinity with water or other polar compounds. It is indicated for the formulation of aqueous or oily gels, providing the thickening and the thixotropy desired. The silica may be hydrophobically changed through reactions, such as that of sililation. The hydrophobic silicas may also perform the function of thickening agents, however they originate less viscous gels and are highly thixotropic. In the experiment below, the behavior of the colloidal silica as to the sensorial and suspensory capacity was studied, according to the following base formulation:

| Ingredients | Essay 1 | Essay 2 | Essay 3 | Essay 4 | Essay 5 |
|---|---|---|---|---|---|
| Silica | 4,0% | 4,5% | 5,00% | 5,5% | 6,0% |
| Mixture of Cosmetic Oils | Qsp. 100% | Qsp. 100% | Qsp. 100% | Qsp. 100% | Qsp. 100% |
| Polyethylene Terephtalate | 0,5% | 0,5% | 0,5% | 0,5% | 0,5% |
| Suspensory Capacity (50° C) | Insufficient decantation over a time less than 24 hours | Insufficient decantation over a time less than 24 hours | Insufficient decantation over a time less than 07 days | Insufficient decantation over a time less than 7 days | Satisfactory. No decantation over a term of 7 days. |
| Sensorial | Easy Dispersion Sensorial Slightly dry Acceptable sliding | Average Dispersion Dry sensorial, Acceptable sliding. | Average Dispersion, Dry sensorial acceptable sliding | Difficult Dispersion, Dry sensorial, low sliding. | Difficult dispersion, extremely dry sensorial extremely low sliding. |

After these preliminary essays, one could ascertain that, by combining the sensorial features, such as easy dispersion, emollient sensorial and a good sliding on the skin and a good suspensory capacity, it would not be possible making use of the silica only. With a minimal amount for obtaining a good suspensory capacity (6,0%), without occurring a separation of phases, the product sensorial was highly affected.

Upon testing a combination of styrene-ethylene/propylene copolymer and that of a hydrophylic silica, one could notice a synergism between the materials for favoring the suspensory capacity, further resulting in an interesting sensorial summation. As the materials exhibit opposite sensorial features, the styrene-ethylene/propylene copolymer keeps the emollient property of the base, however it is sticky at high concentrations. On the other hand, silica reduces the dispersibility making the sensorial drier. The result of the combination exhibited good dispersibility, good sliding on the skin and a good emollient property, with a low stickiness. Further, the satisfactory suspensory capacity was managed with relatively low concentrations of both materials, making evident the synergism of this combination. Ipso facto, the instant invention does not offer only an oil-gel having a texture and pleasant sensorial and unsatisfactory suspensory capacity, or a gel-oil having an exaggerating dry suspensory and sensorial optimum capacity of a difficult dispersibility or a high stickiness. The invention discloses an oil-gel having a pleasant texture, soft touch, emollient and silky, easy dispersibility, transparent, with a low stickiness and with an optimum suspensory capacity, and, thus, enabling the formulation of anhydrous products in form of oil-gel, paste or pomade. It may further rely upon the presence of particles in suspension, with the aim of protection and/or decoration of the formulation and/or of the skin, mucosas and hair, such as iron oxide, mica, polyethylene terephathalate (glitters), silica derivatives, titanium oxide, zinc dioxide or other dispersed solids, as well as the respective mixtures.
In the following example, a copolymer of styrene-ethylene/ propylene and silica combination was tested on an oily basis and its suspensory and sensorial capacity was evaluated:

| INGREDIENTS | ESSAY 1 |
|---|---|
| Styrene-ethylene/propylene copolymer | 6,0% |
| Silica | 2,0% |
| Mixture of cosmetic oils | Qsp. 100% |
| Polyethylene terephthalate | 0.5% |
| Suspensory capacity (50°C) | Satisfactory. No decantation over the term of 7 days. |
| Sensorial | Easy dispersibility, sensorial emollient with a low stickiness. |

With this study, it was concluded that the combination of the thickening agents and oily suspensory copolymers of styrene-ethylene/propylene and silica in the formulation of an oil-gel, paste or pomade shows synergism both for favoring the suspensory capacity and for securing the best sensorial and application features. This combination is, therefore, appropriate for thickening mixtures of components of an oily nature, used in the cosmetic industry, such as mineral oil and fractions thereof and derivatives, hydrocarbons, silicones, esters, ethers and cosmetic ethers, "Guerbert" alcohols, fatty acid triacylglyceral and vegetable oils, inter alia, and mixtures thereof; all these combinations being included in this invention.

The oil-gel obtained from the combination of styrene-ethylene/propylene copolymer in concentrations from 0,1 % to 15% in mass with silica in concentrations of 0,1% to 15% in mass, on a compound basis composed of cosmetic ingredients of an oily nature, constitutes an appropriate vehicle for the suspension of 0,01% to 50% in mass of particles for the protection of the skin or mucosas or decoration of the skin, mucosas or hair, such as iron oxide, mica, polyethylene terephthalate (glitters), silica derivatives, titanium bioxide zinc oxide, inter alia, as well as mixtures thereof.

A fragrance or essential oils from plants may be included for providing a pleasant smell to the product, in concentrations from 0,1% to 10% by weight in respect of the total weight of the composition.
Preservative substances may be included, according to this invention.
All the preservative agents allowed for cosmetic use to this date and mixtures thereof, are included in the scope of this invention, in concentrations from 2% by weight relative to the total weight of the composition.
Vitamins, such as to tocopherol or tocopherol acetate, retinol palmitate or other oily active ingredients, such as antioxidant agents and vegetable extracts, may further be included with the aim of promoting skin treatment.
For protecting the compositions from oxidation, there may be selected antioxidant agents. By way of instances, we may cite hydroxytoluene butyl (BHT), hydroxianisol butyl (BHA), vegetable antioxidants or others, or mixtures thereof.
The obtainment of additional property of a solar protection of the formulation of the invention is reached, in addition to the presence of inorganic pigments through the inclusion of a set of organic photoprotector components, such as p-methoxycyanate of 2-ethylhexyl, butylmethoxidibenzoilmethane, or others, and mixtures thereof, conveniently solubilized in their adequate solvents.
Dyestuff may be added, in order to modify the aspect of the product.
The following example is illustrative of the invention. The materials are combined and the amounts represented in percent by weight, based upon the total weight of the composition:

| | |
|---|---|
| Decorative particles (iron oxide, mica, polyethylene terephthalate, silica derivatives, titanium bioxide or others and mixtures thereof | 0,01%-50% |
| Inorganic solar filters (titanium bioxide or zinc oxide) | 0,01%-50% |
| Fragrance or essential oils | 0,05%-10% |
| Preservative substances (parabenes or others) | 0,01%-2% |
| Vitamins, vegetable extracts, or other solubilized active elements or in suspension | 0,01%-10% |
| Antioxidants (BHT), BHA or others) | 0,01%-0,5% |
| Organic solar filters (p-methoxiacynamate of 2-ethylhexyl,butylmethoxydibenzoilmethane, or others and mixtures thereof) | 0,01%-30% |
| Mineral oil and fractions thereof and derivatives, hydrocarbons, silicones, esters, ethers and cosmetic ethers, "Guerbert" alcohols, fatty acid triacylglyceral and vegetable oils, inter alia, and mixtures thereof | 1%-95% |
| Styrene-ethylene/propylene copolymer | 0,1-15% |
| Silica | 0,1%-15% |
| Surfactants (solubilizers or emulsifiers | 0,01%-10% |
| | |

The instant invention is not limited to the embodiments herein commented on or illustrated, and must be understood in its ample scope. Plenty of modifications and other embodiments of the invention will come to the mind of those skilled in the art to whom this invention belongs, having the benefit of the teaching submitted in the previous descriptions and drawings attached. Moreover, it must be understood that the invention is not restricted to the specific form disclosed and that the modifications and other forms are understood as included within the scope of the claims attached. Even though specific terms are used herein, they have only a generic and descriptive nature and not for the purpose of limitation.

## Claims

1. OILY COSMETIC COMPOSITION HAVING A SUSPENSORY CAPACITY, cosmetic oily composition in the form of oil-gel with lubricating effects, moisturizing, decorative and/or protector of the skin, mucosas and hair, exhibiting excellent suspensory capacity, combined with an easy application, good sliding on the skin and pleasant silky sensorial, **characterized in that** it exhibits basic formulations with contents by weight of product from 0.1% to 15% of a block copolymer composed of styrene-ethylene/propylene and 0,1% to 15% of silica on an oily basis, in synergic association.

2. OILY COSMETIC COMPOSITION HAVING A SUSPENSORY CAPACITY, cosmetic composition of claim 1, **characterized by** the presence of components of an oily nature, such as mineral oils (hydrocarbons), its fractions and derivatives, as well as mixtures thereof.

3. OILY COSMETIC COMPOSITION HAVING A SUSPENSORY CAPACITY, cosmetic composition of claim 1, **characterized by** the combination with other components of an oily nature, such as cosmetic esters and ethers, silicones, elastomers, Guerbert alcohols, vegetable oils and mixtures thereof.

4. OILY COSMETIC COMPOSITION HAVING A SUSPENSORY CAPACITY, cosmetic composition of claim 1, **characterized by** the combination with surfactants, such as emulsifiers or solubilizers.

5. OILY COSMETIC COMPOSITION HAVING A SUSPENSORY CAPACITY, cosmetic composition of claim 1, **characterized by** the presence of preservative substances, such as parabenes, in concentrations from 0,01% to 2% by weight of the product.

6. OILY COSMETIC COMPOSITION HAVING A SUSPENSORY CAPACITY, cosmetic composition of claim 1, **characterized by** the presence of fragrance and/or essential oils from plants in concentrations from 0.1% to 10% by weight of the product.

7. OILY COSMETIC COMPOSITION HAVING A SUSPENSORY CAPACITY, cosmetic composition of claim 1, **characterized by** the presence of the natural or synthetic dyestuff in concentrations from 0.1 % to 10% by weight of the product.

8. OILY COSMETIC COMPOSITION HAVING A SUSPENSORY CAPACITY, cosmetic composition of claim 1, **characterized by** the presence of active ingredients for the treatment or protection of the skin, mucosas and hair, such as vitamins, antioxidants, vegetable extracts, inter alia, in concentrations from 0,01% to 10% by weight of the product.

9. OILY COSMETIC COMPOSITION HAVING A SUSPENSORY CAPACITY, cosmetic composition of claim 1, **characterized by** the presence of antioxidants, such as BHT, BHA and mixtures thereof, in concentrations from 0.01% to 1% by weight of the product.

10. OILY COSMETIC COMPOSITION HAVING A SUSPENSORY CAPACITY, cosmetic composition of claim 1, **characterized by** the presence of organic solar filters UVA and UVB, such as p-Methoxicyanamate of 2-ethylexyl, butylmethoxidibenzoilmethane or others and mixtures thereof, in concentrations from 0,01 % to 20% by weight of the product.

11. OILY COSMETIC COMPOSITION HAVING A SUSPENSORY CAPACITY, cosmetic composition of claim 1, **characterized by** exhibiting transparent, translucent or opaque visual in the form of oil, oil-gel, paste or pomade.

12. OILY COSMETIC COMPOSITION HAVING A SUSPENSORY CAPACITY, cosmetic composition of claim 1, **characterized by** the presence of particles in suspension, such as iron oxide, mica, polyethylene therephathalate (glitters), silica derivatives, titanium dioxide, zinc oxide, inter alia, as well as mixtures thereof.
